# FASCICULE DE BREVET EUROPEEN

(11) **EP 0 704 448 B1**
(45) Date de publication et mention de la délivrance du brevet: **05.12.2001**
(21) Numéro de dépôt: 95402174.7
(22) Date de dépôt: 28.09.1995
(51) Int. Cl.: C07D 491/107, C07D 495/10, C07D 471/10, C07D 491/113, A61K 31/415, A61K 31/435

(54) **Nouvelles imidazolidines substituées par un hétérocycle, leur procédé et des intermédiaires de préparation, leur application comme médicaments et les compositions pharmaceutiques les renfermant**
Heterocyclisch substituierte Imidazolidine, Verfahren und Zwischenprodukte zu ihrer Herstellung, ihre Verwendung als Arzneimittel und diese enthaltende pharmazeutische Zubereitungen
Heterocyclic substituted imidazolidines, process and intermediates for their preparation, their use as medicaments and pharmaceutical compositions containing them

(30) Priorité: 29.09.1994 FR 9411649
(43) Date de publication de la demande: 03.04.1996
(73) Titulaire: Aventis Pharma S.A., 92160 Antony (FR)
(72) Inventeur: Claussner, André, F-93250 Villemomble (FR); Goubet, François, F-75015 Paris (FR); Teutsch, Jean-Georges, F-93500 Pantin (FR)

(56) Documents cités:
- EP-A- 0 578 516
- FR-A- 2 139 925
- FR-A- 2 329 276
- US-A- 5 221 675
- CHEMICAL ABSTRACTS, vol. 75, no. 17, 25 Octobre 1971, Columbus, Ohio, US; abstract no. 110315n, MASASHIRO TORIGOSHI ET AL 'Spirohydantoin derivatives' page 437 ; & JP-A-46 023 738 (YOSHITOMI)
- CHEMICAL ABSTRACTS, vol. 73, no. 19, 9 Novembre 1970, Columbus, Ohio, US; abstract no. 98869v, GIORGIO WINTERS ET AL 'Synthesis of spirohydantoins from basic heterocyclic ketones' page 368 ; & FARMACO, ED. SCI., vol.25, no.9, 1970 pages 681 - 693
- CHEMICAL ABSTRACTS, vol. 66, no. 15, 10 Avril 1967, Columbus, Ohio, US; abstract no. 65473p, MICHIO NAKANISHI ET AL 'Spirohydantoin compounds' page 6157 ; & JP-A-42 001 179 (YOSHITOMI)

## Description

La présente invention concerne de nouvelles imidazolidines substituées par un hétérocycle, leur procédé et des intermédiaires de préparation, leur application comme médicaments et les compositions pharmaceutiques les renfermant.

Dans la demande japonaise J 48087030 sont décrites des 3-phényl 2-thiohydantoïnes qui sont présentées comme inhibant la germination de certaines plantes.

Dans le brevet français 2.329.276 sont décrites des imidazolidines qui sont présentées comme possédant une activité antiandrogène. Les produits de ce brevet sont cependant différents des produits de la présente demande de brevet.

La présente invention a donc pour objet les produits de formule (I) : 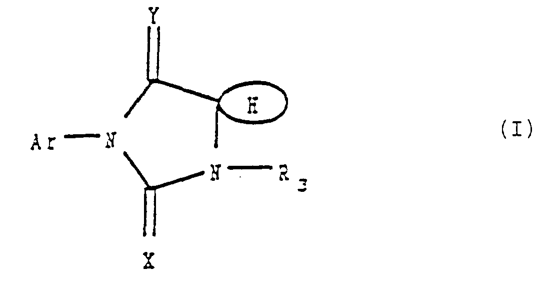 dans laquelle (Aᵣ) représente le radical : 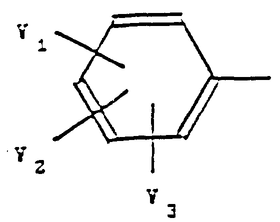 (H) représente le radical : 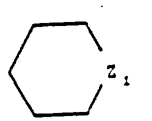
dans lequel Z₁ représente un atome d'oxygène, de soufre éventuellement oxydé sous forme de sulfoxyde ou de sulfone ou un radical -N-R₄ dans lequel R₄ est choisi parmi l'atome d'hydrogène et les radicaux alkyle,
W₁, W₂ et W₃ identiques ou différents sont choisis parmi l'atome d'hydrogène, les atomes d'halogène et les radicaux cyano, nitro, trifluorométhyle, et carboxy libre, estérifié ou salifié,
R₃ est choisi parmi l'atome d'hydrogène et les radicaux alkyle, éventuellement substitués par un ou plusieurs substituants choisis parmi les atomes d'halogène et les radicaux hydroxy, éventuellement salifié ou éthérifié, alcoxy et carboxy libre, estérifié, amidifié ou salifié,
X représente un atome d'oxygène ou de soufre,
Y représente un atome d'oxygène ou un radical NH,
les radicaux alkyle renfermant au plus 4 atomes de carbone,étant entendu que Aᵣ ne représente pas un radical phényle non substitué lorsque R3 représente un atome d'hydrogène,
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

Pour la définition des substituants indiqués ci-dessus et dans ce qui suit, les définitions utilisées peuvent avoir les valeurs suivantes :
le radical hétérocyclique que représente (H) peut être choisi parmi les radicaux des hétérocycles saturés tels que définis ci-dessus, connus de l'homme du métier.

On peut citer à titre d'exemple et de façon non exhaustive :
- d'une part, les radicaux renfermant un atome d'oxygène, d'azote ou de soufre tel que notamment les radicaux : 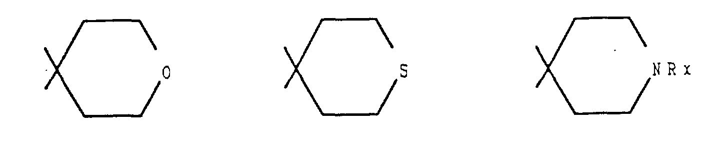 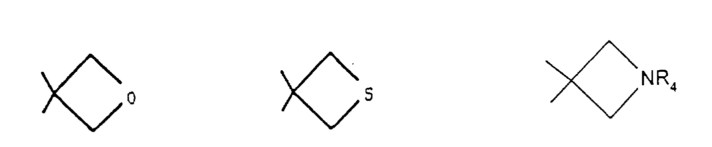
dans lesquels R₄ est choisi parmi l'atome d'hydrogène et les radicaux alkyle, ainsi qu'il est défini ci-dessus et ci-après,

Le terme alkyle désigne un radical alkyle linéaire ou ramifié, ayant au plus 12 atomes de carbone, tel que par exemple les radicaux méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle, heptyle, octyle, décyle, undécyle, dodécyle.

On préfère les radicaux alkyle ayant au plus 4 atomes de carbone et notamment les radicaux méthyle, éthyle, propyle, isopropyle.

Le terme alkényle désigne un radical alkényle linéaire ou ramifié, ayant au plus 12 atomes de carbone tel que par exemple les radicaux vinyle, allyle, 1-propényle, butényle, pentényle, hexenyle.

Parmi les radicaux alkényle, on préfère ceux à 4 atomes de carbone tels que les radicaux allyle, propényle ou butényle.

Le terme alcoxy désigne un radical linéaire ou ramifié, renfermant au plus 12 et de préférence 4 atomes de carbone tel que de préférence les radicaux méthoxy, éthoxy, propoxy ou isopropoxy, mais aussi butoxy linéaire, secondaire ou tertiaire.

Par halogène, on entend bien entendu, les atomes de fluor, de chlore, de brome ou d'iode.

On préfère les atomes de fluor, de chlore ou de brome.

Comme exemples particuliers de radicaux alkyle substitués par un ou plusieurs halogènes ou haloalkyle, on peut citer les radicaux monofluoro, chloro, bromo ou iodométhyle ou -éthyle, difluoro, dichloro ou dibromométhyle, trifluorométhyle.

Comme exemples particuliers de radicaux alcoxy substitués par un ou plusieurs halogènes ou haloalcoxy, on peut citer les radicaux bromoéthoxy, trifluorométhoxy, trifluoroéthoxy ou encore pentafluoroéthoxy.

Comme exemples particuliers, on peut citer de façon non exhaustive ceux dans lesquels le radical phényle est substitué en position ortho, méta ou para, par un ou plusieurs radicaux choisis parmi l'atome de fluor et les radicaux alkylthio, hydroxy, hydroxyalkyle, alcoxy, trifluorométhyle, trifluoroéthyle, pentafluoroéthyle et cyano.

Le ou les radicaux carboxy des produits de formule (I) peuvent être salifiés, amidifiés ou estérifiés par les groupements divers connus de l'homme du métier.

Par carboxy estérifié on entend par exemple les radicaux alkyloxycarbonyle tels que par exemple les radicaux méthoxycarbonyle, éthoxycarbonyle, propoxycarbonyle, n-butyl-, tert-butyloxycarbonyle, ou encore benzyloxycarbonyle, ces radicaux alkyles pouvant être substitués par un ou plusieurs radicaux choisis par exemple parmi les atomes d'halogène, les radicaux hydroxyle, alcoxy, acyle, alcyloxy, alkylthio, amino ou aryle comme, par exemple, dans les groupements chlorométhyle, hydroxypropyle, propionyloxyméthyle, méthylthiométhyle, diméthylaminoéthyle, benzyle ou phénéthyle.

On peut citer des radicaux formés avec les restes esters facilement clivables tels que les radicaux méthoxyméthyle, éthoxyméthyle ; les radicaux acyloxyalkyle tels que pivaloyloxyméthyle, pivaloyloxyéthyle, acétoxyméthyle ou acétoxyéthyle ; les radicaux alkyloxycarbonyloxy alkyle tels que les radicaux méthoxycarbonyloxy méthyle ou éthyle, les radicaux isopropyloxycarbonyloxy méthyle ou éthyle.

Une liste de tels radicaux esters peut-être trouvée par exemple dans le brevet européen EP 0 034 536.

Par carboxy amidifié on entend les groupes du type 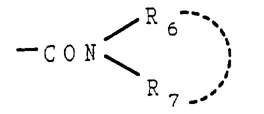 dans lesquels les radicaux R₆ et R₇ identiques ou différents représentent un atome d'hydrogène ou un radical alkyle ayant de 1 à 4 atomes de carbone tels que les radicaux méthyle, éthyle, propyle, isopropyle, butyle, isobutyle, sec-butyle ou tert-butyle.

Dans les groupes 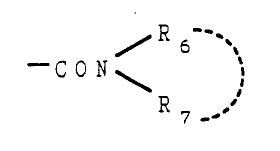 définis ci-dessus, on préfère ceux dans lesquels le radical 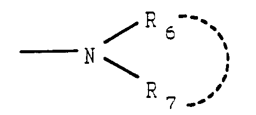 représente le radical amino, mono ou diméthylamino, mono ou diéthylamino, méthyléthylamino, monopropylamino ou monobutylamino. Le radical 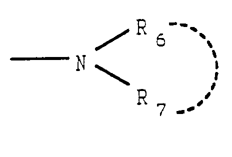 peut également représenter un hétérocycle qui peut ou non comporter un hétéroatome supplémentaire. On peut citer les radicaux pyrrolyle, imidazolyle, indolyle, pipéridino, morpholino, pipérazinyle. On préfère les radicaux pipéridino, morpholino ou pipérazinyle éventuellement substitué ou le second atome d'azote, comme par exemple dans méthylpipérazinyle, fluorométhylpipérazinyle, éthylpipérazinyle, propylpipérazinyle, phénylpipérazinyle ou benzylpipérazinyle : dans ces deux derniers radicaux, les radicaux phényle et benzyle peuvent être substitués, comme par exemple dans chlorophényle ou trifluorophényle.

Par carboxy salifié on entend les sels formés par exemple avec un équivalent de sodium, de potassium, de lithium, de calcium, de magnésium ou d'ammonium. On peut également citer les sels formés avec les bases organiques telles que la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

On préfère le sel de sodium.

Par radical hydroxyle estérifié, éthérifié ou protégé, on entend respectivement les radicaux 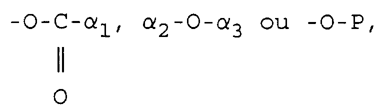 formés à partir d'un radical hydroxyle, selon les méthodes usuelles connues de l'homme du métier et dans lesquels P représente un groupement protecteur,
α₁, α₂ et α₃ représentent un radical alkyle, alkényle, alkynyle, aryle ou arylalkyle, ayant au plus 12 atomes de carbone et éventuellement substitués ainsi qu'il est défini ci-dessus notamment pour R₃.

Des exemples de groupement protecteur P, ainsi que la formation du radical hydroxyle protégé, sont donnés notamment dans le livre usuel de l'homme du métier : Protective Groups in Organic Synthesis, Theodora W. Greene, Harvard University, imprimé en 1981 par Wiley-Interscience Publishers, John Wiley & Sons.

Le groupement de protection du radical hydroxyle que peut représenter P, peut être choisi dans la liste ci-dessous :
par exemple formyle, acétyle, chloroacétyle, bromoacétyle, dichloroacétyle, trichloroacétyle, trifluoroacétyle, méthoxyacétyle, phénoxyacétyle, benzoyle, benzoylformyle, p-nitrobenzoyle. On peut citer également les groupements éthoxycarbonyle, méthoxycarbonyle, propoxycarbonyle, βββ-trichloroéthoxycarbonyle, benzyloxycarbonyle, tert-butoxycarbonyle, 1-cyclo propyléthoxycarbonyle, tétrahydropyrannyle, tétrahydrothiopyrannyle, méthoxytétrahydropyrannyle, trityle, benzyle, 4-méthoxybenzyle, benzhydryle, trichloroéthyle, 1-méthyl 1-méthoxyéthyle, phtaloyle, propionyle, butyryle, isobutyryle, valéryle, isovaléryle, oxalyle, succinyle et pivaloyle, phénylacétyle, phénylpropionyle, mésyle, chlorobenzoyle, para-nitrobenzoyle, para-tert-butylbenzoyle, caprylyle, acryloyle, méthylcarbamoyle, phénylcarbamoyle, naphtylcarbamoyle. P peut notamment représenter le radical 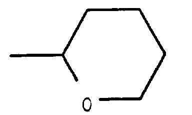 ou encore un dérivé du silicium tel que triméthylsilyle.

Lorsque les produits de formule (I) telle que définie ci-dessus comportent un radical amino salifiable par un acide il est bien entendu que ces sels d'acides font également partie de l'invention.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut citer plus particulièrement les sels formés avec les acides chlorhydrique ou méthanesulfonique par exemple.

Parmi les produits préférés de l'invention, on peut citer plus précisément les produits de formule (I) telle que définie ci-dessus dont les noms suivent :
- le 4-(2,4-dioxo 1-(4-hydroxybutyl) 8-oxa 1,3-diazaspiro (4.5)décan 3-yl) 2-(trifluorométhyl) benzonitrile,
- le 4-(2,4-dioxo 1-(2-fluoroéthyl) 8-oxa 1,3-diazaspiro (4.5)décan 3-yl) 2-(trifluorométhyl) benzonitrile,
- le 3-(4-cyano 3-(trifluorométhyl) phényl) 2,4-dioxo 8-oxa 1,3-diazaspiro(4.5)décane 1-acétonitrile,
- le 4-(2,4-dioxo 1-(4-hydroxybutyl) 8-thia 1,3-diazaspiro (4.5)décan 3-yl) 2-(trifluorométhyl) benzonitrile.
   L'invention a aussi pour objet un procédé de préparation des produits de formule (I) telle que définie ci-dessus, caractérisé en ce que l'on fait agir en présence d'une base tertiaire un produit de formule (II) :

   Ar-N=C=X (II)

   dans laquelle X a la signification indiquée ci-dessus et Ar a la signification indiquée ci-dessus, dans laquelle les éventuelles fonctions réactives sont éventuellement protégées, avec un produit de formule (III) : 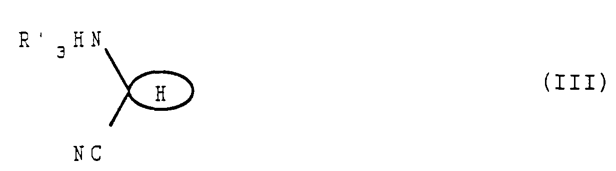
dans laquelle (H) a la signification indiquée ci-dessus et R'₃ a la signification indiquée ci-dessus pour R₃ dans laquelle les éventuelles fonctions réactives sont éventuellement protégées pour obtenir un produit de formule (Ia) : 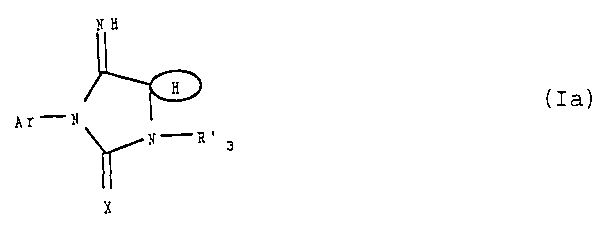 dans laquelle Ar, (H) et R'₃ ont les significations indiquées ci-dessus,
- produits de formule (Ia) ainsi obtenue, telle que définie ci-dessus, que, le cas échéant, et si nécessaire ou si désiré, l'on peut soumettre à l'une quelconque ou plusieurs des réactions suivantes, dans un ordre quelconque :
a) réaction d'élimination des éventuels groupements protecteurs que peut porter R'₃, puis le cas échéant action d'un agent d'estérification, d'amidification ou de salification,
b) réaction d'hydrolyse du groupement >C=NH en fonction carbonyle,
c) transformation du groupement >C=O en groupement >C=S,
d) transformation du ou des groupements >C=S en groupement >C=O,
e) lorsque R'₃ représente un radical alcoxyalkyle, réaction de transformation de R'₃ en radical hydroxyalkyle,
f) lorsque R'₃ représente un atome d'hydrogène, action d'un réactif de formule Hal-R"₃ dans laquelle R"₃ a les valeurs de R'₃ à l'exception de la valeur hydrogène et Hal représente un atome d'halogène pour obtenir des produits de formule (Ia) telle que définie ci-dessus, dans lesquelles R"₃ a la signification indiquée précédemment,
g) si désiré, action sur les produits obtenus en f, d'un agent d'élimination des éventuels groupements protecteurs que peut porter R"₃ ou le cas échéant, action d'un agent d'estérification, d'amidification ou de salification,
h) réaction de salification par un acide minéral ou organique ou par une base pour obtenir le sel correspondant,
i) réaction d'élimination des éventuels groupements protecteurs que peuvent porter les fonctions réactives protégées.

L'action des produits de formule (II) avec les produits de formule (III) ou (X) est effectuée de préférence dans un solvant organique tel que le tétrahydrofuranne ou le dichloroéthane mais on peut également utiliser l'éther éthylique ou l'éther isopropylique.

On opère en présence d'une base tertiaire telle que la triéthylamine ou encore la pyridine ou la méthyléthylpyridine.

Les éventuelles fonctions réactives que peut comporter R₃ et qui sont éventuellement protégées, sont les fonctions hydroxy ou amino. On utilise pour protéger ces fonctions des groupements protecteurs usuels. On peut citer par exemples les groupements protecteurs suivants du radical amino : tert-butyle, tert-amyle, trichloroacétyle, chloroacétyle, benzhydryle, trityle, formyle, benzyloxycarbonyle.

Comme groupement protecteur du radical hydroxy on peut citer les radicaux tels que formyle, chloroacétyle, tétrahydropyrannyle, triméthylsilyle, tert-butyl diméthylsilyle.

Il est bien entendu que la liste ci-dessus n'est pas limitative et que d'autres groupements protecteurs, par exemple connus dans la chimie des peptides peuvent être utilisés. Une liste de tels groupements protecteurs se trouve par exemple dans le brevet français BF 2.499.995 dont le contenu est incorporé ici par référence.

Les réactions éventuelles d'élimination des groupements protecteurs sont effectuées comme indiqué dans ledit brevet BF 2.499.995. Le mode préféré d'élimination est l'hydrolyse acide à l'aide des acides choisis parmi les acides chlorhydrique, benzène sulfonique ou para toluène sulfonique, formique ou trifluoroacétique. On préfère l'acide chlorhydrique.

La réaction éventuelle d'hydrolyse du groupement >C=NH en groupement cétone est également effectuée de préférence à l'aide d'un acide tel que l'acide chlorhydrique aqueux par exemple au reflux.

Lorsque l'hydrolyse du groupement >C=NH en groupement carbonyle est effectuée sur une molécule comportant également un groupement >C=S, celui-ci peut être transformé en groupement >C=O. Le radical OH libre que peut comporter éventuellement R₃ peut être alors transformé en radical SH.

La réaction de transformation du ou des groupements >C=O en groupement >C=S est effectuée à l'aide du réactif dit de Lawesson de formule : 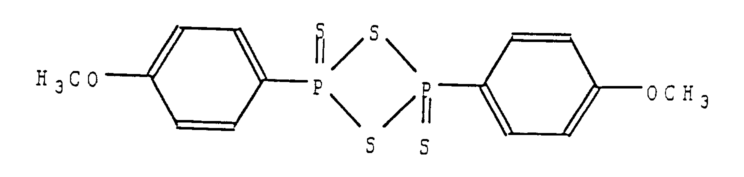 qui est un produit commercialisé par exemple par la firme FLUKA et dont l'utilisation est décrite par exemple dans la publication : Bull. Soc. Chim. Belg. vol 87, N° 3, (1987) p. 229.

Lorsque l'on veut transformer deux fonctions >C=O en deux fonctions >C=S on opère en présence d'un excès de réactif de Lawesson. Il en est de même lorsque l'on part d'une molécule comportant une fonction >C=S et une fonction >C=O et que l'on veut transformer ladite fonction >C=O en fonction >C=S.

Par contre lorsque l'on part d'une molécule comportant deux fonctions >C=O et que l'on veut obtenir un produit ne comportant qu'une seule fonction >C=S, on opère en présence d'un déficit de réactif de Lawesson. On obtient alors en général un mélange de trois produits : chacun des deux produits comportant une fonction >C=O et une fonction >C=S et le produit comportant deux fonctions >C=S. Ces produits peuvent être ensuite séparés par les méthodes usuelles telles que la chromatographie.

L'action sur le produit de formule (Ia) du réactif de formule Hal-R"₃ est effectuée en présence d'une base forte telle que l'hydrure de sodium ou de potassium. On peut opérer par réaction de transfert de phase en présence de sels d'ammonium quaternaires tels que le tert-butyl ammonium.
- Les groupements protecteurs que peut porter le substituant R"₃ pouvant être par exemple un de ceux précédemment cités pour R₃. Les réactions d'élimination des groupements protecteurs s'effectuent dans les conditions indiquées ci-dessus.

L'élimination du groupement terbutyldiméthylsilyle peut être fait par exemple au moyen de l'acide chlorhydrique.
- L'estérification éventuelle des produits de formule (I), telle que définie ci-dessus, dans laquelle R"₃ comporte un radical OH libre est effectuée dans des conditions classiques. On peut utiliser par exemple un acide ou un dérivé fonctionnel, par exemple un anhydride tel que l'anhydride acétique en présence d'une base telle que la pyridine.

L'estérification ou la salification éventuelle des produits de formule (I) telle que définie ci-dessus, dans laquelle R"₃ représente un groupement COOH est effectuée dans les conditions classiques connues de l'homme du métier.
- L'amidification éventuelle des produits de formule (I), telle que définie ci-dessus, dans laquelle R"₃ comporte un radical COOH est effectuée dans des conditions classiques. On peut utiliser une amine primaire ou secondaire sur un dérivé fonctionnel de l'acide par exemple un anhydride symétrique ou mixte.

La présente invention a également pour objet un procédé de préparation de produits de formule (I') : 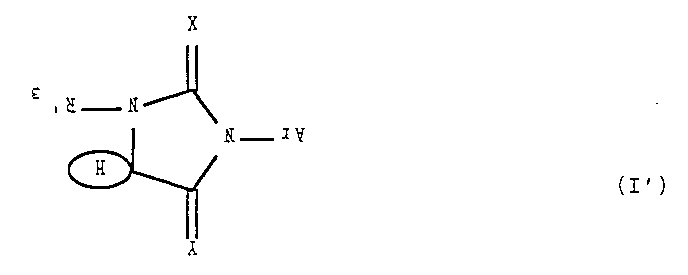 dans laquelle Ar, X, Y, R'₃ et (H) ont les significations indiquées ci-dessus, caractérisé en ce que l'on fait réagir un produit de formule (VIII) :

Ar-Hal (VIII)

dans laquelle Ar a la signification précédente et Hal représente un atome d'halogène avec un produit de formule (IX) : 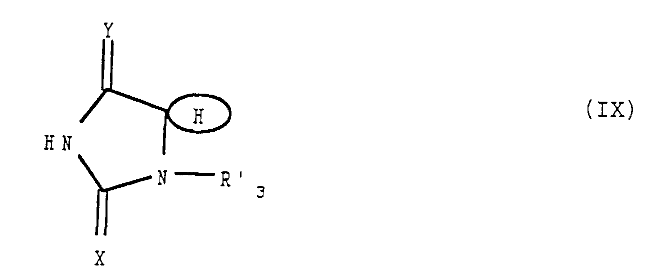 dans laquelle X, Y, R'₃ et (H) ont les significations indiquées ci-dessus, la réaction s'effectuant en présence d'un catalyseur et éventuellement d'un solvant.

En ce qui concerne les produits de formule (VIII), le terme Hal désigne de préférence l'atome de chlore, mais peut aussi représenter un atome de brome ou d'iode.

Le rôle du catalyseur est vraisemblablement de piéger l'halogénure d'hydrogène qui se dégage et ainsi de faciliter la réaction de condensation du produit de formule (VIII) avec le produit de formule (IX) pour donner le produit recherché.

L'invention a plus précisément pour objet un procédé tel que défini ci-dessus dans lequel le catalyseur est un métal sous forme native ou oxydée ou une base.

Quand le catalyseur utilisé est un métal, ce métal peut être du cuivre ou du nickel. Il peut être sous forme native, sous forme d'oxyde métallique ou encore sous forme de sels métalliques.

Les sels métalliques peuvent être un chlorure ou un acétate.

Quand le catalyseur est une base, cette base peut être par exemple la soude ou la potasse et on peut, si désiré, ajouter au milieu réactionnel du diméthylsulfoxyde.

L'invention a plus précisément pour objet un procédé tel que défini ci-dessus dans lequel le catalyseur est choisi parmi l'oxyde cuivreux, l'oxyde cuivrique, le cuivre sous forme native et une base telle que la soude ou la potasse.

Le cuivre sous forme native utilisé comme catalyseur est préférentiellement sous forme de poudre.

L'invention a particulièrement pour objet un procédé tel que défini ci-dessus dans lequel le catalyseur est l'oxyde cuivreux.

Le solvant utilisé est préférentiellement choisi parmi des éthers à haut point d'ébullition tels que, par exemple, l'oxyde de phényle, le diglyme, le triglyme et le diméthylsulfoxyde mais peut être également, par exemple, une huile à haut point d'ébullition telle que la paraffine ou la vaseline.

L'invention a plus particulièrement pour objet un procédé tel que défini ci-dessus caractérisé en ce que l'on opère en présence d'un solvant de type éther tel que l'oxyde de phényle, le diglyme, le triglyme ou le diméthylsulfoxyde.

L'invention a tout particulièrement pour objet un procédé tel que défini ci-dessus dans lequel le solvant utilisé est l'oxyde de phényle ou le triglyme.

Le procédé de préparation du produit recherché défini ci-dessus peut être réalisé sous pression ou à la pression atmosphérique, à une température préférentiellement élevée.

L'invention a ainsi pour objet un procédé tel que défini ci-dessus caractérisé en ce que la réaction est réalisée à une température supérieure à 100°C et de préférence supérieure à 150°C.

L'invention a plus précisément pour objet un procédé tel que défini ci-dessus caractérisé en ce que la réaction est réalisée pendant plus de 2 heures.

L'invention a très précisément pour objet un procédé tel que défini ci-dessus caractérisé en ce que la réaction est réalisée en présence d'oxyde cuivreux, dans le triglyme, à une température supérieure ou égale à 200°C et pendant plus de 3 heures.

Les produits objet de la présente invention possèdent d'intéressantes propriétés pharmacologiques, notamment ils se fixent sur le récepteur des androgènes et ils présentent une activité anti-androgénique.

Des tests donnés dans la partie expérimentale illustrent ces propriétés.

Ces propriétés rendent les produits de formule (I) telle que définie ci-dessus, de la présente invention utilisables comme médicaments principalement pour :
- le traitement des adénomes et des néoplasies de la prostate ainsi que dans l'hypertrophie bénigne de la prostate, seul ou en association avec des analogues de la LHRH. Ils peuvent également être utilisés dans le traitement de tumeurs bénignes ou malignes possédant des récepteurs aux androgènes et plus particulièrement les cancers du sein, de la peau, des ovaires, de la vessie, du système lymphatique, du rein et du foie,
- le traitement d'affections cutanées tel que l'acné, l'hyperséborrhée, l'alopécie ou l'hirsutisme. Ces produits peuvent donc être utilisés en dermatologie seuls ou en association avec des antibiotiques tels que les dérivés des acides azélaique et fusidique, l'érythromycine, ainsi que des dérivés de l'acide rétinoïque ou un inhibiteur de la 5α-réductase tel que le (5α, 17β)-1,1-diméthyléthyl 3-oxo 4-aza-androst-1-ène 17-carboxamide (ou Finastéride, Merck 11ème ed.) pour le traitement de l'acné, l'alopécie ou l'hirsutisme. Ils peuvent également être associés à un produit stimulant la croissance des cheveux tel que le Minoxidil pour le traitement de l'alopécie.

Les produits de formule (I) telle que définie ci-dessus, peuvent également être utilisés dans le domaine vétérinaire pour le traitement de troubles comportementaux tel que l'agressivité, d'affections androgénodépendantes, tel que le circum analum chez le chien et de tumeurs présentant des récepteurs aux androgènes. Ils peuvent également être utilisés pour provoquer une castration chimique chez l'animal.

Les produits de formule (I), telle que définie ci-dessus, sous forme radioactive (tritium, carbone 14, iode 125 ou fluor 18) peuvent encore être utilisés comme marqueur spécifique des récepteurs aux androgènes. Ils peuvent aussi être utilisés en diagnostic en imagerie médicale.

L'invention a donc pour objet l'application, à titre de médicaments, des produits de formule (I) telle que définie ci-dessus, pharmaceutiquement acceptables et particulièrement des produits de formule (F) telle que définie ci-dessus, pharmaceutiquement acceptables.

L'invention a plus particulièrement pour objet l'application, à titre de médicaments, des produits suivants :
- le 4-(2,4-dioxo 1-(4-hydroxybutyl) 8-oxa 1,3-diazaspiro (4.5)décan 3-yl) 2-(trifluorométhyl) benzonitrile,
- le 4-(2,4-dioxo 1-(2-fluoroéthyl) 8-oxa 1,3-diazaspiro (4.5)décan 3-yl) 2-(trifluorométhyl) benzonitrile,
- le 3-(4-cyano 3-(trifluorométhyl) phényl) 2,4-dioxo 8-oxa 1,3-diazaspiro(4.5)décane 1-acétonitrile,
- le 4-(2,4-dioxo 1-(4-hydroxybutyl) 8-thia 1,3-diazaspiro (4.5)décan 3-yl) 2-(trifluorométhyl) benzonitrile.

Les produits peuvent être administrés par voie parentérale, buccale, perlinguale, rectale ou topique.

L'invention a aussi pour objet les compositions pharmaceutiques, caractérisées en ce qu'elles renferment, à titre de principe actif, un au moins des médicaments tels que définis ci-dessus.

Ces compositions peuvent être présentées sous forme de solutions ou de suspensions injectables, de comprimés, de comprimés enrobés, de capsules, de sirops, de suppositoires, de crèmes, de pommades, de gels et de lotions. Ces compositions peuvent également être présentées sous forme de liposomes. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, le beurre de cacao, les corps gras d'origine animale ou végétale, les dérivés paraffiniques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'homme, par voie orale.

Les produits de formule (II) utilisés au départ de l'invention peuvent être obtenus par action du phosgène lorsque X représente un atome d'oxygène ou du thiophosgène lorsque X représente un atome de soufre sur l'amine correspondante de formule (A) :

Ar-NH₂ (A)

Un exemple d'une telle préparation est donné dans le brevet européen EP 0494819. Lorsque Ar représente un radical phényle, un produit de ce type est décrit également dans le brevet français BF 2.329.276. et des amines de formule (A) sont décrites dans le brevet européen EP 0.002.892 ou le brevet français BF 2.142.804.

Les produits de formules (VIII) et (IX), utilisés au départ du procédé indiqué ci-dessus, pour l'obtention de produits de formule (I'), telle que définie ci-dessus, sont connus et disponibles dans le commerce ou peuvent être préparés selon des méthodes connues de l'homme de métier.

La préparation de produits de formule (IX) est décrite notamment dans les publications suivantes :
- Zhur. Préklad. Khim. 28, 969-75 (1955) (CA 50, 4881a, 1956)
- Tétrahédron 43, 1753 (1987)
- J. Org. Chem. 52, 2407 (1987)
- Zh. Org. Khim. 21, 2006 (1985)
- J. Fluor. Chem. 17, 345 (1981)
ou dans les brevets :
- allemand DRP 637.318 (1935)
- européen EP 0.130.875
- japonais JP 81.121.524.

Les produits de formule (IX) qui sont des dérivés de l'hydantoïne sont largement utilisés et cités dans la littérature comme par exemple dans les articles suivants :
- J. Pharm. Pharmacol., 67, Vol. 19(4), p. 209-16 (1967)
- Khim. Farm. Zh., 67, Vol. 1 (5) p. 51-2
- Brevet allemand 2.217.914
- Brevet européen 0.091.596
- J. Chem. Soc. Perkin. Trans. 1, p. 219-21 (1974).

Les produits de formule (III) telle que définie ci-dessus peuvent être trouvés dans le commerce, tels que notamment ceux utilisés dans la préparation des exemples décrite ci-après. On peut citer par exemple :
le 4-amino-tétrahydro-2H-pyran-4-carbonitrile
le 4-amino tétrahydro-2H-thiopyran 4-carbonitrile
le 4-amino 1-méthyl pipéridine 4-carbonitrile.

Les exemples suivants illustrent l'invention sans toutefois la limiter.

### EXEMPLE 1 : 4-(4-imino 2-oxo 8-oxa 1,3-diazaspiro[4.5]decan 3-yl) 2-(trifluorométhyl) benzonitrile

### STADE 1 : 4-amino-tétrahydro-2H-pyran-4-carbonitrile

On introduit successivement 8 ml d'ammoniaque 1,58 g de chlorure d'ammonium 1,23 g de cyanure de sodium, refroidit la solution obtenue au bain de glace méthanol à ≃ -7°C et ajoute à une température ≤ 0°C, 2 ml de tétrahydro-4H-pyran 4-one, laisse remonter à température ambiante et agite vigoureusement 18 h. On extrait 3 fois au chlorure de méthylène, lave à l'eau salée et sèche. On obtient 2,49 g de produit attendu (cristaux translucides). F ≃ 46-47°C.
Analyses physiques :
I.R. : (CHCl₃) cm⁻¹

| | |
|---|---|
| NH₂ | 3390-3374-3324 |
| C≡N | 2225 |
| NH₂ déf. | 1605 |

### STADE 2 : 4-(4-imino 2-oxo 8-oxa 1,3-diazaspiro[4.5]decan 3-yl) 2-(trifluorométhyl) benzonitrile

On introduit 883 mg du produit obtenu au stade 1 ci-dessus dans 7 ml de 1,2-dichloroéthane et 0,3 ml de triéthylamine.

On amène à ≃ -7°C et ajoute goutte à goutte en 15 minutes 6,4 ml du produit obtenu à la préparation de l'exemple 7 de la demande de brevet européen EP 0494819 puis laisse remonter à température ambiante.

On laisse une heure, évapore à sec et purifie sur silice avec pour éluant chlorure de méthylène-acétone : 7/1. On obtient ainsi 1,85 g de produit attendu (cristaux blancs)
F = 249-250°C.
Analyses physiques :
I.R. : (nujol) cm⁻¹

| | |
|---|---|
| OH/NH | 3340-3295 |
| C≡N | 2240 |
| C=O | 1750 |
| Système conjugué | 1678-1612-1572-1508 |
| aromatique | |

### EXEMPLE 2 : 4-(2,4-dioxo 8-oxa 1,3-diazaspiro[4.5]decan 3-yl) 2-(trifluorométhyl) benzonitrile

On introduit 1,66 g du produit de l'exemple 1 dans 30 ml de méthanol, 10 ml de chloroforme, 10 ml d'acide chlorhydrique 2N, et porte à reflux pendant 50 minutes, ajoute 100 ml d'eau et extrait 3 fois à l'acétate d'éthyle. On lave la phase organique à l'eau salée, sèche et purifie sur silice avec pour éluant chlorure de méthylène-acétone : 85-15. On dissout dans 100 ml d'isopropanol à ≃ 70°C, filtre et concentre, glace 1 heure, essore et sèche. On obtient ainsi 1,425 g de produit attendu (cristaux blancs). F = 192-193°C.
Analyses physiques :
I.R. : (nujol) cm⁻¹
Adsorption OH/NH

| | |
|---|---|
| C≡N | 2240 |
| C=O | 1790-1732 |
| Aromatiques | 1614-1582-1506 |

### EXEMPLE 3 : 4-(2,4-dioxo 1-(4-hydroxybutyl) 8-oxa 1,3-diazaspiro[4.5]decan 3-yl) 2-(trifluorométhyl) benzonitrile

On introduit 55 mg d'hydrure de sodium à 50 %, ajoute goutte à goutte en 25 minutes, 340 mg du produit de l'exemple 2 et 25 ml de diméthylsulfoxyde. On rince avec 0,5 ml de diméthylsulfoxyde et 20 minutes après cessation du dégagement d'hydrogène on ajoute 0,41 g de 4-iodobutoxy triméthylsilane, et laisse agir 18 h à température ambiante.

On verse alors sur 10 ml d'eau et extrait 4 fois à l'éther. On lave la phase organique à l'eau puis à l'eau salée, sèche et reprend par le mélange 10 ml de méthanol et 1 ml d'acide chlorhydrique 2N. Après 30 minutes, on verse sur 20 ml de chlorure de sodium solution saturée et extrait 3 fois au chloroforme et sèche. On purifie sur silice avec pour éluant chlorure de méthylène-acétone 8-2 et obtient 369 mg de produit attendu (mousses friables blanches).
Analyses physiques :
I.R. : (CHCl₃) cm⁻¹

| | |
|---|---|
| OH | 3626-3485 |
| C≡N | 2235 |
| C=O | 1775-1721 |
| Aromatiques | 1615-1602-1577-1505 |

### EXEMPLE 4 : 4-(2,4-dioxo 1-(2-hydroxyéthyl) 8-oxa 1,3-diazaspiro[4.5]decan 3-yl) 2-(trifluorométhyl) benzonitrile

On introduit 82 mg d'hydrure de sodium à 50 %, 510 mg du produit de l'exemple 2, 4 ml de diméthylsulfoxyde, rince avec 0,5 ml de diméthylsulfoxyde et 20 mn après cessation de dégagement d'hydrogène, on ajoute en 1 fois, 572 mg de 2-iodoéthoxy diméthylterbutyl silane et porte la solution à 40°C 1 h 30 puis 18 h à température ambiante. On verse sur 40 ml d'eau contenant ≃ 0,2 g de phosphate monopotassique et extrait 4 fois à l'éther, on lave à l'eau puis à l'eau salée, sèche. On purifie sur silice avec pour éluant chlorure de méthylène-acétate d'éthyle. On reprend par 16 ml de méthanol, 3 ml d'acide chlorhydrique 2N et chauffe à 40°C pendant 40 mn. On verse sur 50 ml de bicarbonate de sodium au 1/2, extrait 3 fois au chloroforme, lave à l'eau salée et sèche. On purifie sur silice avec pour éluant chlorure de méthylène-acétone : 8-2 et obtient 451 mg de produit attendu (cristaux blancs). F = 196-197°C.
Analyses physiques :
I.R. : (nujol) cm⁻¹

| | |
|---|---|
| OH/NH | 3450 |
| C=N | 2240 |
| >=O | 1775-1718 |
| Aromatiques | 1615-1576-1508 |

### EXEMPLE 5 : 4-(2,4-dioxo 1-(3-hydroxypropyl) 8-oxa 1,3-diazaspiro[4.5]decan 3-yl) 2-(trifluorométhyl) benzonitrile

On procède comme à l'exemple 4, à partir de 109 mg d'hydrure de sodium au 1/2, 510 mg du produit de l'exemple 2 et 4 ml de diméthylsulfoxyde et rince avec 0,5 ml de diméthylsulfoxyde puis 20 mn après cessation du dégagement d'hydrogène on ajoute 1,004 g de 3-bromopropoxydiphényl tert-butyl silane et porte la solution à ≃ 50°C. En procédant comme à l'exemple 4, on reprend ensuite par 30 ml de méthanol, 10 ml d'acide chlorhydrique 2N, 10 ml de chloroforme et porte à reflux.

Après purification sur silice avec pour éluant chlorure de méthylène-acétone : 8-2, on obtient 441 mg de produit attendu (cristaux blancs). F = 155-156°C.
Analyses physiques :
I.R. : (nujol) cm⁻¹
Adsorption générale NH/OH

| | |
|---|---|
| C≡N | 2240 |
| >=O | 1778-1714 |
| Aromatiques | 1618-1580-1539-1511 |

### EXEMPLE 6 : 3-(4-cyano 3-(trifluorométhyl) phényl) 2,4-dioxo 8-oxa 1,3-diazaspiro[4.5]decane 1-butanoate d'éthyle

On procède comme à l'exemple 3 à partir de 123 mg d'hydrure de sodium à 50 %, 510 mg du produit de l'exemple 2, 3,5 ml de diméthylsulfoxyde, rince avec 0,5 ml de diméthylsulfoxyde et 15 mn après cessation du dégagement d'hydrogène, ajoute 454 mg de 4-bromobutyrate d'éthyle et porte la solution à 40°C pendant 50 mn. On verse sur 40 ml d'eau contenant 0,4 g de phosphate monopotassique, extrait 4 fois à l'éther, lave la phase organique à l'eau puis à l'eau salée, sèche et purifie sur silice avec pour éluant chlorure de méthylène-acétate d'éthyle : 85-15. On obtient ainsi 584 mg de produit attendu (cristaux blancs). F = 129-130°C.
Analyses physiques :
I.R. : (CHCl₃) cm⁻¹

| | |
|---|---|
| C≡N | 2230 |
| >=O | 1776-1722 |
| Aromatiques | 1614-1575-1505 |

### EXEMPLE 7 : Acide 3-(4-cyano 3-(trifluorométhyl) phényl) 2,4-dioxo 8-oxa 1,3-diazaspiro[4.5]decane 1-butanoique

On introduit 522 mg du produit de l'exemple 6, 20 ml de méthanol, chauffe à 30°C, ramène à température ambiante puis ajoute 2 ml de soude 2N et laisse 4 h 30 puis verse sur 30 ml d'H₂O et amène à pH 2-3. On extrait 3 fois à l'acétate d'éthyle, lave la phase organique à l'eau puis à l'eau salée, sèche et purifie sur silice avec pour éluant chlorure de méthylène-méthanol : 9-1, puis concrétise par trituration dans 0,2 ml d'isopropanol, éther 5 ml. On obtient ainsi 345 mg de produit attendu (cristaux blancs). F = 151-152°C. Analyses physiques :
I.R. : (nujol) cm⁻¹
Adsorption complexe NH/OH

| | |
|---|---|
| C≡N | 2240 |
| >=O | 1780-1720 |
| Aromatiques | 1615-1581-1510 |

### EXEMPLE 8 : 3-(4-méthoxyphényl) 8-oxa 1,3-diazaspiro-[4.5]decane 2,4-dione

On procède comme au stade 2 de l'exemple 1 à partir de 380 mg du produit obtenu au stade 1 de l'exemple 1, 4 ml de 1,2-dichloroéthane et 0,25 ml de triéthylamine et amène à -5°C puis ajoute goutte à goutte 450 mg de 4-méthoxyphényl isocyanate, rince avec 1 ml dans 1,2-dichloroéthane et laisse remonter à température ambiante. Après 1 h, on essore, sèche puis reprend par 20 ml de méthanol, 6 ml de chloroforme, 6 ml d'acide chlorhydrique 2N et porte la solution obtenue au reflux pendant 2 h. On concentre, reprend avec 30 ml d'eau, extrait 5 fois à l'acétate d'éthyle, lave la phase organique au bicarbonate de sodium au 1/2, puis à l'eau salée, sèche et purifie sur silice avec pour éluant chlorure de méthylène-acétone : 85-15. On obtient 493 mg de produit attendu (cristaux blancs). F = 253-254°C.
Analyses physiques :
I.R. : (nujol) cm⁻¹
Adsorption région OH/NH

| | |
|---|---|
| C=O | 1774-1715 |
| Aromatiques | 1609-1590-1518. |

### EXEMPLE 9 : 4-(2,4-dioxo 1-(2-fluoroéthyl) 8-oxa 1,3-diazaspiro[4.5]decan 3-yl) 2-(trifluorométhyl) benzonitrile

On introduit 55 mg d'hydrure de sodium 50 %, ajoute goutte à goutte en ≃ 20 mn, 340 mg du produit de l'exemple 2 et 3 ml de diméthylsulfoxyde et rince avec 0,2 ml de diméthylsulfoxyde. 20 mn après cessation du dégagement d'hydrogène, on ajoute 0,1 ml de 1-bromo 2-fluoroéthane et chauffe à 50°C pendant 1 h 20. On verse sur 10 ml d'eau glacée, contenant 0,2 g de phosphate mono potassique et extrait 5 fois à l'éther. On lave la phase organique à l'eau puis à l'eau salée et sèche. On purifie sur silice avec pour éluant chlorure de méthylène-acétone : 85-15, sèche et cristallise dans l'isopropanol. On obtient ainsi 281 mg de produit attendu (cristaux blancs). F = 192-193°C.
Analyses physiques :
I.R. : (CHCl₃) cm⁻¹

| | |
|---|---|
| C=O | 1777-1723 |
| C≡N | 2238 |
| Aromatiques | 1616-1576-1505 |

### EXEMPLE 10 : 3-(4-cyano 3-(trifluorométhyl) phényl) 2,4-dioxo 8-oxa 1,3-diazaspiro[4.5]decan 1-acétonitrile

On introduit 55 mg d'hydrure de sodium à 50 % et ajoute goutte à goutte en 20 mn 340 mg du produit de l'exemple 2, 2,5 ml de diméthylsulfoxyde et rince avec 0,5 ml de diméthylsulfoxyde. 15 mn après cessation de dégagement d'hydrogène, on ajoute 0,1 ml de bromoacétonitrile et laisse 1 h. On verse sur 15 ml d'eau contenant ≃ 0,3 g de phosphate monopotassique, extrait 3 fois à l'acétate d'éthyle, lave à l'eau puis à l'eau salée, sèche et purifie sur silice avec pour éluant chlorure de méthylène-acétone 85-15. On dissout la résine obtenue dans le mélange chlorure de méthylène 15 ml, isopropanol 30 ml à ≃40°C et concentre jusqu'à cristallisation. On obtient ainsi 267 mg de produit attendu (cristaux blancs). F = 210-211°C.
Analyses physiques :
I.R. : (nujol) cm⁻¹

| | |
|---|---|
| C≡N | 2238 |
| >=O | 1782-1730 |
| Aromatiques | 1612-1570-1504 |

### EXEMPLE 11 : 3-(4-méthylthiophényl) 8-oxa 1,3-diazaspiro-[4.5]décane 2,4-dione

On procède comme à l'exemple 8, à partir de 380 mg de 4-méthylthiophénylisocyanate, 4 ml de 1,2-dichloroéthane et 0,15 ml de triéthylamine.

On obtient ainsi 513 mg de produit attendu. F = 256-257°C.
I.R. : (nujol) cm⁻¹
Adsorption région OH/NH

| | |
|---|---|
| >=O | 1776-1720 |
| Aromatiques | 1500 |

### EXEMPLE 12 : 4-(4-imino 2-oxo 8-thia 1,3-diazaspiro[4.5]decan 3-yl) 2-(trifluorométhyl) benzonitrile

### STADE 1 : 4-amino tétrahydro-2H-thiopyran 4-carbonitrile

On procède comme au stade 1 de l'exemple 1 à partir de 8 ml d'ammoniaque, 1,58 g de chlorure d'ammonium, 1,23 g de cyanure de sodium, 2,51 g de tétrahydro 4H-thiopyran 4-one et agite 18 h à température ambiante. On extrait 3 fois au chlorure de méthylène, lave à l'eau salée et sèche. On obtient ainsi 2,88 g de produit attendu (cristaux blancs). F = 51-52°C.
Analyses physiques :
I.R. : (CHCl₃) cm⁻¹

| | |
|---|---|
| NH₂ | 3398-3381-3321 |
| NH₂ déf | 1617-1584 |
| C≡N | 2225 |

### STADE 2 : 4-(4-imino 2-oxo 8-thia 1,3-diazaspiro[4.5]decan 3-yl) 2-(trifluorométhyl) benzonitrile

On procède comme au stade 2 de l'exemple 1, à partir de 1,42 g du produit obtenu au stade 1 ci-dessus, 14 ml de 1,2-dichloroéthane, 0,5 ml de triéthylamine, porte à une température comprise entre -10°C et -5°C en ≃ 20 mn et ajoute 9,1 ml du produit obtenu à la préparation de l'exemple 7 de la demande de brevet européen EP 0494819 et laisse remonter à température ambiante. On laisse 1 h 20, sèche et purifie sur silice avec pour éluant chlorure de méthylène-acétone : 9-1. On obtient ainsi 2,49 g de produit attendu (cristaux blancs). F = 226-227°C.
Analyses physiques :
I.R. : (nujol) cm⁻¹

| | |
|---|---|
| NH | 3330-3250-3210-3170-3120 |
| C≡N | 2240 |
| C=O | 1750 |
| C=N | 1675-1658 |
| Aromatiques | 1612-1572-1510. |

### EXEMPLE 13 : 4-(2,4-dioxo 8-thia 1,3-diazaspiro[4.5]decan 3-yl) 2-(trifluorométhyl) benzonitrile

On procède comme à l'exemple 2, à partir de 2,34 g du produit de l'exemple 12, 25 ml de méthanol, 5 ml de chloroforme, ajoute 4,5 ml d'acide chlorhydrique 2N et porte à reflux 1 h. Après purification sur silice avec pour éluant chlorure de méthylène-acétone : 95-5. On recristallise dans l'isopropanol. On obtient ainsi 368 mg de produit attendu (cristaux blancs). F = 209-210°C.
Analyses physiques :
I.R. : (nujol) cm⁻¹

| | |
|---|---|
| OH/NH | 3340 |
| C≡N | 2245 |
| >C=O | 1781-1736 |
| Aromatiques | 1612-1576-1508. |

### EXEMPLE 14 : 4-(2,4-dioxo 1-(4-hydroxybutyl) 8-thia 1,3-diazaspiro[4.5]decan 3-yl) 2-(trifluorométhyl) benzonitrile

On procède comme à l'exemple 3 à partir de 55 mg d'hydrure de sodium à 50 %, ajoute goutte à goutte en 20 mn, 355 mg du produit de l'exemple 13, 2,5 ml de diméthylsulfoxyde et rince avec 0,5 ml de diméthylsulfoxyde. 20 mn après cessation du dégagement d'hydrogène, on ajoute 0,41 g de 4-iodo butoxy triméthylsilane. On verse alors sur 25 ml d'eau contenant 0,1 g de phosphate monopotassique, extrait 3 fois à l'éther, lave la phase organique à l'eau puis à l'eau salée et sèche. On reprend par 10 ml de méthanol, 1 ml d'acide chlorhydrique 2N, laisse 30 mn, verse sur 30 ml de chlorure de sodium au 1/2 et extrait 3 fois à l'acétate d'éthyle, puis lave à l'eau salée et sèche. On purifie sur silice avec pour éluant chlorure de méthylène-acétone : 9-1 et obtient ainsi 317 mg de produit attendu (cristaux blancs). F = 139-140°C. Analyses physiques :
I.R. : (CHCl₃) cm⁻¹

| | |
|---|---|
| OH | 3628 |
| C≡N | 2235 |
| >C=O | 1774-1722 |
| Aromatiques | 1615-1601-1505. |

### EXEMPLE 15 : 4-(2,4-dioxo 8,8-dioxydo 8-thia 1,3-diazaspiro[4.5]decan 3-yl) 2-(trifluorométhyl) benzonitrile

On introduit 355 mg du produit de l'exemple 12, 4 ml de chlorure de méthylène et ajoute en 20 mn entre 22 et 25°C, 990 mg d'acide métachloroperbenzoïque, 20 ml de chlorure de méthylène, et rince avec 1 ml de chlorure de méthylène. On laisse 1 h, ajoute 15 ml de thiosulfate de sodium et agite vigoureusement 10 mn. On ajoute 15 ml de bicarbonate de sodium, solution saturée, extrait 3 fois à l'acétate d'éthyle, lave à l'eau puis à l'eau salée et sèche. On purifie sur silice avec pour éluant chlorure de méthylène-acétone : (95-5) puis cristallise dans l'isopropanol. On obtient ainsi 365 mg de produit attendu (cristaux blancs). F = 257-258°C. Analyses physiques :
I.R. : (nujol) cm⁻¹

| | |
|---|---|
| C≡N | 2235 |
| >=O | 1790-1735 |
| SO₂ | 1297-1133 |
| Aromatiques | 1613-1575-1508. |

### EXEMPLE 16 : 4-(2,4-dioxo 8-oxydo 8-thia 1,3-diazaspiro-[4.5]decan 3-yl) 2-(trifluorométhyl) benzonitrile

### (isomère "A")

On introduit 355 mg du produit de l'exemple 13, 20 ml de méthanol, 4 ml de solution aqueuse de métapériodate de sodium et porte à reflux. On évapore le méthanol, reprend par 20 ml d'eau et extrait 4 fois à l'acétate d'éthyle, lave la phase organique à l'eau salée et sèche. On purifie sur silice avec pour éluant chlorure de méthylène-méthanol : 95-5 et recueille 900 mg de sulfoxyde isomère "A". On recristallise dans l'acétone et obtient 292 mg de produit attendu dit isomère "A". F = 260-277°C.
Analyses physiques :
I.R. : (nujol) cm⁻¹
Adsorption générale NH/OH

| | |
|---|---|
| C≡N | 2235 |
| >=O | 1789-1725 |
| S-->0 | 1010 |
| Aromatiques | 1612-1572-1509. |

### EXEMPLE 17 : 4-(2,4-dioxo 8-oxydo 8-thia 1,3-diazaspiro-[4.5]decan 3-yl) 2-(trifluorométhyl) benzonitrile

### (isomère "B")

On procède comme à l'exemple 16 et purifie sur silice avec pour éluant chlorure de méthylène-méthanol 9-1.

On obtient 80 mg de sulfoxyde isomère "B" que l'on recristallise dans l'acétone et obtient ainsi 72 mg de produit attendu dit isomère "B". F = 295°C.
Analyses physiques :
I.R. : (nujol) cm⁻¹
Adsorption NH/OH

| | |
|---|---|
| C≡N | 2240 |
| >C=O | 1797-1730 |
| S--O | 1015 |
| Aromatiques | 1618-1580-1509. |

### EXEMPLE 18 : 3-(4-cyano 3-(trifluorométhyl) phényl) 2,4-dioxo 8-thia 1,3-diazaspiro[4.5]decane 1-butanoate d'éthyle

On procède comme à l'exemple 6, à partir de 96 mg d'hydrure de sodium 50 % et introduit en ≃ 20 mn, 390 mg du produit de l'exemple 13, 2,5 ml de diméthylsulfoxyde et rince avec 0,5 ml de diméthylsulfoxyde. 20 mn après la cessation de dégagement d'hydrogène, on ajoute 370 mg de 4-bromobutyrate d'éthyle et porte la solution à 40°C pendant 1 heure. On verse alors sur 15 ml d'eau glacée contenant 0,1 g de phosphate monopotassique et extrait 4 fois à l'éther, lave la phase organique à l'eau puis à l'eau salée et sèche. On purifie sur silice avec pour éluant chlorure de méthylène-acétate d'éthyle : 95-5 et obtient 449 mg de produit attendu (mousses friables blanches).
Analyses physiques :
I.R. : (CHCl₃) cm⁻¹

| | |
|---|---|
| C≡N | 2236 |
| >=O | 1774-1723 |
| Aromatiques | 1615-1575-1505. |

### EXEMPLE 19 : Acide 3-(4-cyano 3-(trifluorométhyl) phényl) 2,4-dioxo 8-thia 1,3-diazaspiro[4.5]décane 1-butanoique

On procède comme à l'exemple 7 à partir de 440 mg du produit de l'exemple 18, 20 ml de méthanol, 2 ml de soude 2N, agite 3 h 30 à température ambiante et ajoute 3 ml d'acide chlorhydrique 2N. On purifie sur silice avec pour éluant chlorure de méthylène-méthanol : 95-5 et obtient 346 mg de produit attendu (cristaux blancs). F = 197-198°C.
Analyses physiques :
I.R. : (nujol) cm⁻¹

| | |
|---|---|
| C≡N | 2235 |
| >=O | 1770-1725 |
| Acide | 1710 |
| Aromatiques | 1615-1575-1505. |

### EXEMPLE 20 : 4-(4-imino 8-méthyl 2-thioxo 1,3,8-triazaspiro-[4.5]decan 3-yl) 2-(trifluorométhyl) benzonitrile

### STADE 1 : 4-amino 1-méthyl pipéridine 4-carbonitrile

On procède comme au stade 1 de l'exemple 1, à partir de 8 ml d'ammoniaque, 1,58 g de chlorure d'ammonium, 1,23 g de cyanure de sodium et ajoute 2,5 ml de 1-méthylpipéridone. On agite 18 h à température ambiante, extrait 3 fois au chloroforme, lave la phase organique à l'eau salée et sèche. On obtient ainsi 2,41 g de produit attendu (sirop jaune orangé).
Analyses physiques :
I.R. : (CHCl₃) cm⁻¹

| | |
|---|---|
| NH₂ | 3378-3320 |
| C≡N | 2226 |
| NH₂ déf. | 1602 |

### STADE 2 : 4-(4-imino 8-méthyl 2-thioxo 1,3,8-triazaspiro-[4.5]decan 3-yl) 2-(trifluorométhyl) benzonitrile

On procède comme au stade 2 de l'exemple 1, à partir de 140 mg du produit obtenu au stade 1 ci-dessus, 1,5 ml de 1,2-dichloroéthane, 0,1 ml de triéthylamine et ajoute goutte à goutte 230 mg du produit obtenu à la préparation de l'exemple 11 de la demande de brevet européen EP 0494819 et 1,5 ml de 1,2-dichloroéthane. On laisse 2 h, évapore à sec et purifie sur silice avec pour éluant chlorure de méthylène-acétone : 8-2. On obtient ainsi 315 mg de produit attendu (cristaux ivoire). F > 264°C.
Analyses physiques :
I.R. : (nujol) cm⁻¹

| | |
|---|---|
| NH | ≃ 3190 |
| C≡N | 2240 |
| >=NH | 1700-1688-1678 |
| Aromatiques | 1618-1580-1518-1505 |

### EXEMPLE 21 : 4-(8-méthyl 4-oxo 2-thioxo 1,3,8-triazaspiro-[4.5]decan 3-yl) 2-(trifluorométhyl) benzonitrile

On introduit 300 mg du produit de l'exemple 20, 6 ml de méthanol, 1,5 ml d'acide chlorhydrique 2N et porte à reflux pendant une heure puis ramène à température ambiante, verse sur 20 ml d'ammoniaque au 1/2, sature de chlorure de sodium, extrait 3 fois à l'acétate d'éthyle et sèche. On purifie sur silice avec pour éluant chlorure de méthylène-méthanol : 9-1. On obtient ainsi 260 mg de produit attendu (cristaux blancs). F = 229-230°C.
Analyses physiques :
I.R. : (nujol) cm⁻¹

| | |
|---|---|
| OH/NH | 3330-3340 + associé |
| C≡N | 2236 |
| C=O | 1757 |
| Aromatiques | 1614-1580-1514-1502. |

### EXEMPLE 22 : 4-(4-imino 8-méthyl 2-oxo 1,3,8-triazaspiro-[4.5]decan 3-yl) 2-(trifluorométhyl) benzonitrile

On procède comme au stade 2 de l'exemple 20, à partir de 153 mg du produit obtenu au stade 1 de l'exemple 20, 1,5 ml de 1,2-dichloroéthane et 0,1 ml de triéthylamine, place dans un bain d'eau glacée, ajoute 1 ml du produit obtenu à la préparation de l'exemple 7 de la demande de brevet européen EP 0494819 et laisse remonter à température ambiante. On laisse 1 h, sèche et purifie sur silice avec pour éluant chlorure de méthylène-méthanol : 7-3. On obtient ainsi 277 mg de produit attendu (cristaux blancs). F = 199-200°C.
Analyses physiques :
I.R. : (nujol) cm⁻¹

| | |
|---|---|
| NH | 3290-3240-3130 |
| C≡N | 2224 |
| C=O | 1746 |
| C=NH | 1677-1669 |
| Aromatiques | 1609-1568-1512. |

### EXEMPLE 23 : 4-(2,4-dioxo 8-méthyl 1,3,8-triazaspiro[4.5]-decan 3-yl) 2-(trifluorométhyl) benzonitrile

On procède comme à l'exemple 2 à partir de 3 g du produit de l'exemple 22, 70 ml de méthanol, 17 ml d'acide chlorhydrique 2N et porte à reflux pendant 1 h 30. On verse sous 200 ml d'ammoniaque + 100 g de glace saturé en chlorure de sodium, extrait 4 fois à l'acétate d'éthyle, lave à l'eau salée, sèche et purifie sur silice avec pour éluant chlorure de méthylène-méthanol : 9-1. Après cristallisation dans chlorure de méthylène-éther isopropylique, on obtient 2,12 g de produit attendu (cristaux blancs). F = 188-189°C.
Analyses physiques :
I.R. : (CHCl₃) cm⁻¹

| | |
|---|---|
| =C-NH | 3445 |
| C≡N | 2230 |
| >=O | 1789-1729 |
| Aromatiques | 1615-1576-1505. |

### EXEMPLE 24 : 4-(8,8-diméthyl) 2,4-dioxo 7,9-dioxa 1,3-diazas-piro[4.5]décan 3-yl) 2-(trifluorométhyl) benzonitrile

### STADE 1 : 1,3 bis [(tétrahydro 2H pyran-2 yl)oxy]2 propanone

On introduit 9 g de 2,5-dihydroxy 1,4-dioxane 2,5-diméthanol dans 60 ml de dioxanne et porte la suspension à environ 70°C pendant 15 minutes puis ramène à la température ambiante. On ajoute alors 20 ml de 3,4-dihydro 2H-pyran et 300 mg d'acide paratoluène sulfonique monohydraté et maintient la température à environ 40°C puis laisse une nuit à température ambiante.

On verse alors sur un mélange de 300 ml de bicarbonate de sodium solution saturée + 10 ml de triéthylamine et extrait 4 fois au chlorure de méthylène. On lave la phase organique à l'eau salée et sèche.

Après purification par passage sur silice avec pour éluant cycloacétate d'éthyle/triéthylamine : 8/2, on obtient 17 g de produit attendu (sirop jaune pâle).
ANALYSES :
IR CHCl3 (cm⁻¹)
Absence OH

| | |
|---|---|
| O=C | 1736 |

### STADE 2 : 2-amino 3-((tétrahydro-2H-pyran-2-yl) oxy) 2-(((tétrahydro-2H-pyran-2-yl) oxy) méthyl) propanenitrile

On introduit 5,6 g du produit obtenu au stade 1 ci-dessus dans 8 ml d'ammoniaque, amène à environ -5°C et ajoute successivement 1,58 g de chlorure d'ammonium et 1,23 g de cyanure de sodium et laisse remonter à température ambiante environ 40 minutes puis chauffe à 40°C ± 5°C sous agitation pendant une nuit. On ramène à température ambiante et extrait 3 fois au chloroforme, lave la phase organique à l'eau salée et sèche.

Après purification sur silice avec pour éluant cycloacétate d'éthyle/triéthylamine : 3/7, on obtient 4,41 g de produit attendu (sirop jaune pâle)
ANALYSES :
IR CHCl3 (cm⁻¹)

| | |
|---|---|
| -CN | 2235 |
| NH2 | 3390-3317 |

### STADE 3 : 4-(5-imino-2-oxo-4,4-bis(((tétrahydro-2H-pyran-2-yl) oxy) méthyl) 1-imidazolidinyl) 2-(trifluorométhyl) benzonitrile

On introduit 570 mg du produit obtenu au stade 2 ci-dessus dans 5 ml d'éther isopropylique et 0,28 ml de triéthylamine et amène à -30°C puis ajoute en une heure une solution de 1,2-dichloroéthane à 18,4 g %g et 2,32 g du produit obtenu à la préparation 1.

On ajoute 4 ml de chlorure de méthylène puis laisse remonter à température ambiante, laisse environ 2 heures et sèche. Après purification sur silice avec pour éluant chlorure de méthylène/acétone 9/1, on obtient 700 mg de produit attendu.
ANALYSES :
IR CHCl3 (cm⁻¹)

| | |
|---|---|
| NH | 3442-3317 |
| -CN | 2235 |
| C=O | 1757 |
| C=N | 1670 |
| Aromatique | 1614-1575-1505 |

### STADE 4 : 4-(4,4-bis(hydroxyméthyl)-2,5-dioxo-1-imidazolidinyl)-2-(trifluorométhyl)-benzonitrile

On introduit 300 mg du produit obtenu au stade 3 ci-dessus dans 3 ml de méthanol et 1,5 ml d'acide chlorhydrique 2N et porte à reflux 1h30.

On ramène à température ambiante, verse sur 5 ml de bicarbonate, extrait 4 fois à l'acétate d'éthyle puis lave au chlorure de sodium solution saturée et sèche.

On purifie sur silice avec pour éluant chlorure de méthylène-méthanol : 9/1.

On reprend dans 20 ml d'isopropanol à reflux puis concentre et obtient 225 mg de produit attendu (cristaux blancs)
F : 207-208°C.
ANALYSES :
IR NUJOL (cm⁻¹)

| | |
|---|---|
| OH/NH | 3525-3365-3250 |
| CN | 2240 |
| C=O | 1778-1738 |
| Aromatique | 1618-1578-1506 |

### STADE 5 : 4-(8,8-diméthyl) 2,4-dioxo 7,9-dioxa 1,3-diazaspiro[4.5]décan 3-yl) 2-(trifluorométhyl) benzonitrile

On introduit 0,329 g du produit obtenu au stade 4 ci-dessus, 0,5 ml de 2,2-diméthoxy-propane, 0,02 g d'acide paratoluènesulfonique, 5 ml d'acétone et laisse sous agitation pendant 4 heures puis hydrolyse avec une solution de bicarbonate de sodium et extrait à l'acétate d'éthyle. Les phases organiques sont réunies, lavées à l'eau, séchées, filtrées et concentrées. On chromatographie sur silice avec pour éluant le chlorure de méthylène-acétone : 95-5.

On recristallise dans l'isopropanol et obtient 0,240 g de produit attendu (cristaux blancs) F= 203°C.
ANALYSES :
IR NUJOL (cm⁻¹)

| | |
|---|---|
| CN | 2240 |
| C=O | 1782-1730 |
| Aromatiques | 1615-1580-1507 |

### EXEMPLE 25 :

On a préparé des comprimés ayant la composition suivante:
- Produit de l'exemple 3 100 mg
- Excipient q.s. pour'un comprimé terminé à 300 mg (Détail de l'excipient : lactose, amidon, talc, stéarate de magnésium).

### ETUDE PHARMACOLOGIQUE DES PRODUITS DE L'INVENTION

### Etude de l'affinité des produits de l'invention pour le récepteur androgène

Des rats mâles Sprague Dawley EOPS de 180-200 g, castrés de 24 heures, sont sacrifiés, les prostates prélevées, pesées et homogénéisées à 0°C à l'aide d'un potter verre-verre, dans une solution tamponnée (Tris 10mM, saccharose 0,25M, PMSF (phénylméthanesulfonylfluoride) 0,1mM, Molybdate de sodium 20mM, HCl pH 7,4 ; auxquels on ajoute extemporanément 2mM de DTT (DL dithiothreitol), à raison de 1 g de tissu pour 8 ml de tampon.

L'homogénat est ensuite ultracentrifugé à 0°C, 30 minutes à 209 000 g. Des aliquotes du surnageant obtenu (=cytosol), sont incubées 30 minutes et 24 heures à 0°C, avec une concentration constante (T) de Testostérone tritiée et en présence de concentrations croissantes (0 à 2500.10⁻⁹M), soit de testostérone froide, soit des produits à tester. La concentration de Testostérone tritiée liée (B) est ensuite mesurée dans chaque incubat par la méthode d'adsorption au charbon-dextran.

### Calcul de l'affinité relative de liaison (ARL).

On trace les 2 courbes suivantes : le pourcentage de l'hormone tritiée liée B/T en fonction du logarithme de la concentration de l'hormone de référence froide et B/T en fonction du logarithme de la concentration du produit froid testé.
On détermine la droite d'équation I₅₀=(B/Tmax + B/Tmin)/2. B/T max= % de l'hormone tritiée liée pour une incubation de cette hormone tritiée à la concentration (T).
B/T min= % de l'hormone tritiée liée pour une incubation de cette hormone tritiée à la concentration (T) en présence d'un grand excès d'hormone froide (2500.10⁻⁹M).

Les intersections de la droite I₅₀ et des courbes, permettent d'évaluer les concentrations de l'hormone de référence froide (CH) et du produit froid testé (CX) qui inhibent de 50 % la liaison de l'hormone tritiée sur le récepteur. L'affinité relative de liaison (ARL) du produit testé est déterminé par l'équation ARL=100 (CH)/(CX).
On obtient les résultats suivants exprimés en ARL.

| Produit de référence (Testostérone) : 100 | |
|---|---|
| Produit des exemples | ARL : Incubation 24 heures |
| 3 | 6 |
| 10 | 5 |

## Revendications

1. Produits de formule (I) : 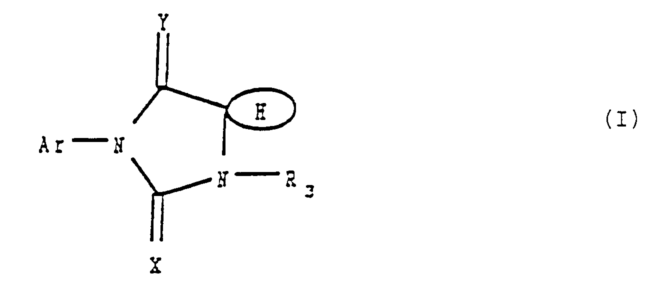 dans laquelle (Aᵣ) représente le radical : 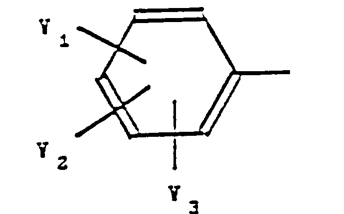 (H) représente le radical : 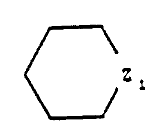
dans lequel Z₁ représente un atome d'oxygène, de soufre éventuellement oxydé sous forme de sulfoxyde ou de sulfone ou un radical -N-R₄ dans lequel R₄ est choisi parmi l'atome d'hydrogène et les radicaux alkyle,
W₁, W₂ et W₃ identiques ou différents sont choisis parmi l'atome d'hydrogène, les atomes d'halogène et les radicaux cyano, nitro, trifluorométhyle, et carboxy libre, estérifié ou salifié,
R₃ est choisi parmi l'atome d'hydrogène et les radicaux alkyle, éventuellement substitués par un ou plusieurs substituants choisis parmi les atomes d'halogène et les radicaux hydroxy, éventuellement salifié ou éthérifié, alcoxy et carboxy, amidifié ou salifié,
X représente un atome d'oxygène ou de soufre,
Y représente un atome d'oxygène ou un radical NH,
les radicaux alkyle renfermant au plus 4 atomes de carbone,étant entendu que Aᵣ ne représente pas un radical phényle non substitué lorsque R3 représente un atome d'hydrogène,
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

2. Les produits suivants :
- le 4-(2,4-dioxo 1-(4-hydroxybutyl) 8-oxa 1,3-diazaspiro (4.5)décan 3-yl) 2-(trifluorométhyl) benzonitrile,
- le 4-(2,4-dioxo 1-(2-fluoroéthyl) 8-oxa 1,3-diazaspiro (4.5)décan 3-yl) 2-(trifluorométhyl) benzonitrile,
- le 3-(4-cyano 3-(trifluorométhyl) phényl) 2,4-dioxo 8-oxa 1,3-diazaspiro(4.5)décane 1-acétonitrile,
- le 4-(2,4-dioxo 1-(4-hydroxybutyl) 8-thia 1,3-diazaspiro (4.5)décan 3-yl) 2-(trifluorométhyl) benzonitrile.

3. Procédé de préparation des produits de formule (I) telle que définie à la revendication 1, **caractérisé en ce que** l'on fait agir en présence d'une base tertiaire un produit de formule (II) :
Ar-N=C=X (II)
dans laquelle X a la signification indiquée à la revendication 1 et Ar a la signification indiquée à la revendication 1 dans laquelle les éventuelles fonctions réactives sont éventuellement protégées,
avec un produit de formule (III) : 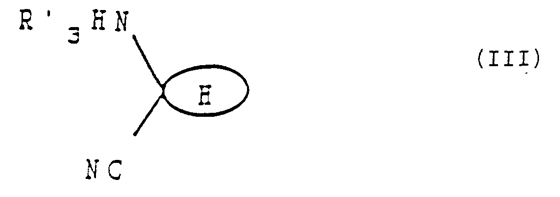 dans laquelle (H) a la signification indiquée à la revendication 1 et R'₃ a la signification indiquée à la revendication 1 pour R₃ dans laquelle les éventuelles fonctions réactives sont éventuellement protégées pour obtenir un produit de formule (Ia) : 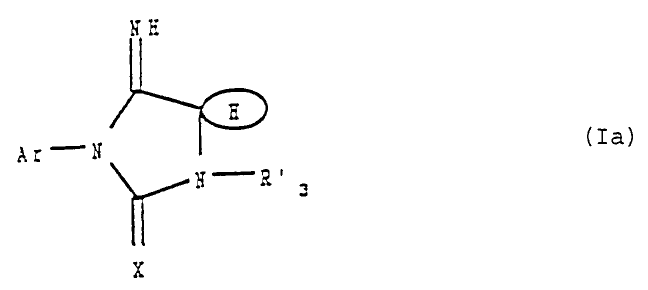 dans laquelle Ar, (H) et R'₃ ont les significations indiquées ci-dessus,
produits de formule (Ia) ainsi obtenue, telle que définie ci-dessus,
que, le cas échéant, et si nécessaire ou si désiré, l'on peut soumettre à l'une quelconque ou plusieurs des réactions suivantes, dans un ordre quelconque :
a) réaction d'élimination des éventuels groupements protecteurs que peut porter R'₃, puis le cas échéant action d'un agent d'estérification, d'amidification ou de salification,
b) réaction d'hydrolyse du groupement >C=NH en fonction carbonyle,
c) transformation du groupement >C=O en groupement >C=S,
d) transformation du ou des groupements >C=S en groupement >C=O,
e) lorsque R'₃ représente un radical alcoxyalkyle, réaction de transformation de R'₃ en radical hydroxyalkyle,
f) lorsque R'₃ représente un atome d'hydrogène, action d'un réactif de formule Hal-R"₃ dans laquelle R"₃ a les valeurs de R'₃ à l'exception de la valeur hydrogène et Hal représente un atome d'halogène pour obtenir des produits de formule (Ia), telle que définie ci-dessus, dans laquelle R"₃ a la signification indiquée précédemment,
g) si désiré, action sur les produits obtenus en f, d'un agent d'élimination des éventuels groupements protecteurs que peut porter R"₃ ou le cas échéant, action d'un agent d'estérification; d'amidification ou de salification,
h) réaction de salification par un acide minéral ou organique ou par une base pour obtenir le sel correspondant,
i) réaction d'élimination des éventuels groupements protecteurs que peuvent porter les fonctions réactives protégées.

4. Procédé de préparation de produits de formule (I') : 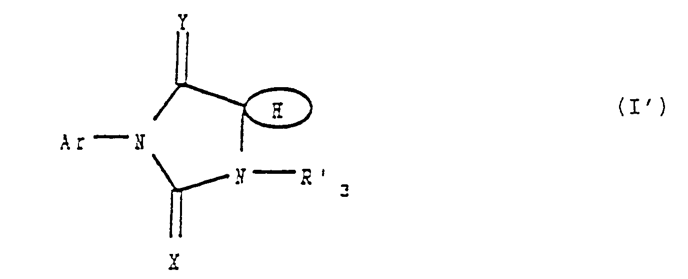 dans laquelle Ar, X, Y, R'₃ et (H) ont les significations indiquées à la revendication 1, **caractérisé en ce que** l'on fait réagir un produit de formule (VIII) :
Ar-Hal (VIII)
dans laquelle Ar a la signification précédente et Hal représente un atome d'halogène avec un produit de formule (IX) : 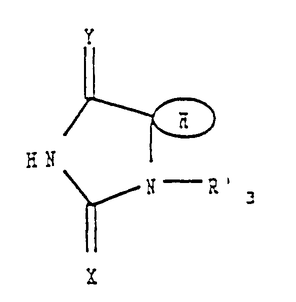 dans laquelle X, Y, R'₃ et (H) ont les significations indiquées à la revendication 1.

5. A titre de médicaments, les produits de formule (I) telle que définie à la revendication 1, pharmaceutiquement acceptables.

6. A titre de médicaments, les produits suivants :
- le 4-(2,4-dioxo 1-(4-hydroxybutyl) 8-oxa 1,3-diazaspiro (4.5)décan 3-yl) 2-(trifluorométhyl) benzonitrile,
- le 4-(2,4-dioxo 1-(2-fluoroéthyl) 8-oxa 1,3-diazaspiro (4.5)décan 3-yl) 2-(trifluorométhyl) benzonitrile,
- le 3-(4-cyano 3-(trifluorométhyl) phényl) 2,4-dioxo 8-oxa 1,3-diazaspiro(4.5)décane 1-acétonitrile,
- le 4-(2,4-dioxo 1-(4-hydroxybutyl) 8-thia 1,3-diazaspiro (4.5)décan 3-yl) 2-(trifluorométhyl) benzonitrile.

7. Les compositions pharmaceutiques contenant, à titre de principe actif, l'un au moins des médicaments tels que définis à l'une quelconque des revendications 5 et 6.

## Patentansprüche

1. Produkte der Formel (I) 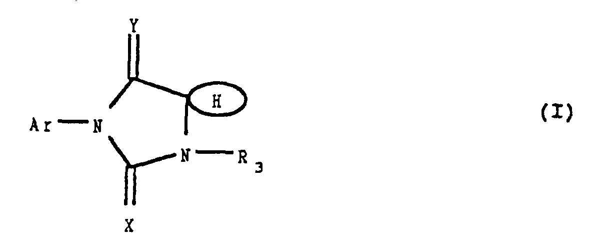 worin (Aᵣ) den Rest 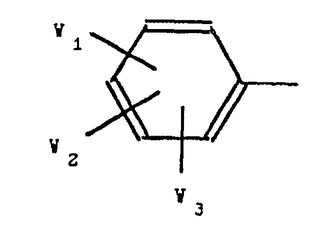 bedeutet, (H) den Rest 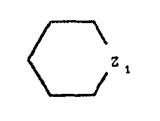
wiedergibt, worin Z₁ ein Sauerstoffatom, ein gegebenenfalls in Form des Sulfoxids oder des Sulfons oxidiertes Schwefelatom oder einen Rest -N-R₄ bedeutet, worin R₄ unter einem Wasserstoffatom und den Alkylresten ausgewählt ist,
W₁, W₂ und W₃, identisch oder verschieden, unter einem Wasserstoffatom, den Halogenatomen und den Cyano-, Nitro-, Trifluormethyl- und den freien, veresterten oder in ein Salz überführten Carboxyresten ausgewählt sind,
R₃ unter einem Wasserstoffatom und den Alkylresten, gegebenenfalls substituiert durch einen oder mehrere Substituenten, ausgewählt unter den Halogenatomen und den Hydroxyresten, gegebenenfalls in ein Salz überführt oder verethert, Alkoxy und Carboxy in freier, veresterter, amidierter oder in ein Salz überführter Form, ausgewählt ist,
X für ein Sauerstoff- oder Schwefelatom steht,
Y ein Sauerstoffatom oder einen Rest NH wiedergibt,
wobei die Alkylreste höchstens 4 Kohlenstoffatome aufweisen, mit der Maßgabe, daß Aᵣ keinen unsubstituierten Phenylrest bedeutet, wenn R₃ ein Wasserstoffatom wiedergibt,
wobei die Produkte der Formel (I) in sämtlichen ihrer möglichen racemischen, enantiomeren und diastereomeren isomeren Formen vorliegen können, sowie die Additionssalze mit Mineral- oder organischen Säuren oder mit den mineralischen und organischen Basen der Produkte der Formel (I).

2. Die folgenden Produkte:
4-(2,4-Dioxo-1-(4-hydroxybutyl)-8-oxa-1,3-diazaspiro[4.5]-decan-3-yl)-2-(trifluormethyl)-benzonitril
4-(2,4-Dioxo-1-(2-fluorethyl)-8-oxa-1,3-diazaspiro[4.5]-decan-3-yl)-2-(trifluormethyl)-benzonitril,
3-(4-Cyano-3-(trifluormethyl)-phenyl)-2,4-dioxo-8-oxa-1,3-diazaspiro[4.5]decan-1-acetonitril
4-(2,4-Dioxo-1-(4-hydroxybutyl)-8-thia-1,3-diazaspiro[4.5]-decan-3-yl)-2-(trifluormethyl)-benzonitril.

3. Verfahren zur Herstellung der Produkte der Formel (I), wie in Anspruch 1 definiert, **dadurch gekennzeichnet, daß** man in Gegenwart einer tertiären Base ein Produkt der Formel (II):
Ar-N=C=X (II)
worin X die in Anspruch 1 angegebene Bedeutung besitzt und Ar die in Anspruch 1 angegebene Bedeutung besitzt, worin die etwaigen reaktiven Funktionen gegebenenfalls geschützt sind, mit einem Produkt der Formel (III): 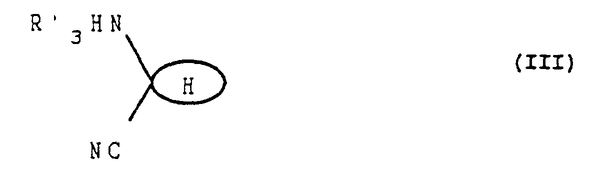 umsetzt, worin (H) die in Anspruch 1 angegebene Bedeutung besitzt und R'₃ die in Anspruch 1 für R₃ angegebene Bedeutung besitzt, worin die etwaigen reaktiven Funktionen gegebenenfalls geschützt sind, um zu einem Produkt der Formel (Ia): 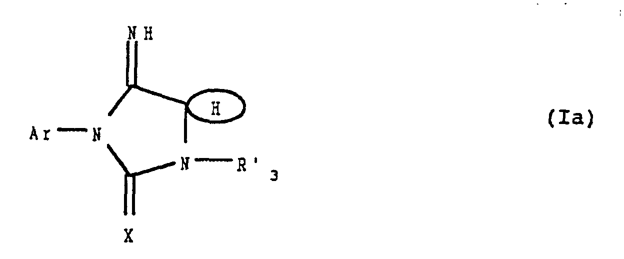 zu gelangen, worin Ar, (H) und R'₃ die vorstehend .angegebenen Bedeutungen besitzen,
die so erhaltenen Produkte der Formel (Ia), wie vorstehend definiert, man gegebenenfalls und erforderlichenfalls oder gewünschtenfalls einer oder mehreren der folgenden Reaktionen
in beliebiger Reihenfolge unterziehen kann:
a) Reaktion zur Eliminierung etwaiger Schutzgruppen, die R'₃ aufweisen kann, hiernach gegebenenfalls Umsetzung mit einem Veresterungsmittel, Amidierungsmittel oder Salzbildungsmittel,
b) Reaktion zur Hydrolyse der Gruppe >C=NH zu einer Carbonylfunktion,
c) Umwandlung der Gruppe >C=O in die Gruppe >C=S,
d) Umwandlung der Gruppe(n) >C=S in die Gruppe >C=O,
e) wenn R'₃ einen Alkoxyalkylrest bedeutet, Reaktion zur Umwandlung von R'₃ in den Hydroxyalkylrest,
f) wenn R'₃ ein Wasserstoffatom bedeutet, die Umsetzung mit einem Reagens der Formel Hal-R"₃, worin R"₃ die Bedeutungen von R'₃ mit Ausnahme der Bedeutung Wasserstoff besitzt und Hal für ein Halogenatom steht, um zu den Produkten der Formel (Ia), wie vorstehend definiert, zu gelangen, worin R"₃ die vorstehend angegebene Bedeutung besitzt,
g) gewünschtenfalls Umsetzung der unter f erhaltenen Produkte mit einem Mittel zur Eliminierung etwaiger Schutzgruppen, die R"₃ aufweisen kann, oder gegebenenfalls Umsetzung mit einem Veresterungs-, Amidierungs- oder Salzbildungsmittel,
h) Reaktion zur Salzbildung mit einer Mineral- oder organischen Säure oder mit einer Base, um zu dem entsprechenden Salz zu gelangen,
i) Umsetzung zur Eliminierung etwaiger Schutzgruppen, die die geschützten reaktiven Funktionen tragen können.

4. Verfahren zur Herstellung der Produkte der Formel (I'): 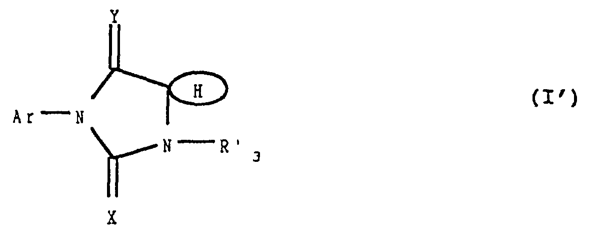 worin Ar, X, Y, R'₃ und (H) die in Anspruch 1 angegebenen Bedeutungen besitzen, **dadurch gekennzeichnet, daß** man ein Produkt der Formel (VIII):
Ar-Hal (VIII)
worin Ar die vorstehende Bedeutung besitzt und Hal für ein Halogenatom steht, mit einem Produkt der Formel (IX): 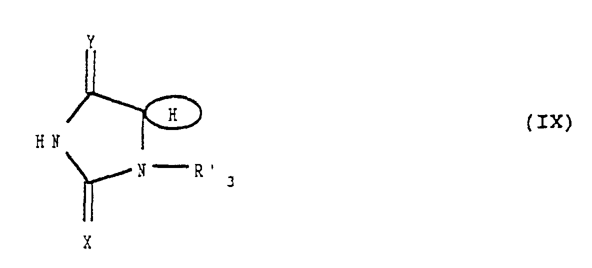 umsetzt, worin X, Y, R'₃ und (H) die in Anspruch 1 angegebenen Bedeutungen besitzen.

5. Als Arzneimittel die pharmazeutisch verträglichen Produkte der Formel (I), wie in Anspruch 1 definiert.

6. Als Arzneimittel die folgenden Produkte:
4-(2,4-Dioxo-1-(4-hydroxybutyl)-8-oxa-1,3-diazaspiro[4.5]-decan-3-yl)-2-(trifluormethyl)-benzonitril,
4-(2,4-Dioxo-1-(2-fluorethyl)-8-oxa-1,3-diazaspiro[4.5]decan-3-yl)-2-(trifluormethyl)-benzonitril,
3-(4-Cyano-3-(trifluormethyl)-phenyl)-2,4-dioxo-8-oxa-1,3-diazaspiro[4.5]decan-1-acetonitril,
4-(2,4-Dioxo-1-(4-hydroxybutyl)-8-thia-1,3-diazaspiro-[4.5]decan-3-yl)-2-(trifluormethyl)-benzonitril.

7. Pharmazeutische Zusammensetzungen, enthaltend als Wirkstoff zumindest eines der wie in einem der Ansprüche 5 und 6 definierten Arzneimittel.

## Claims

1. Products of formula (I): 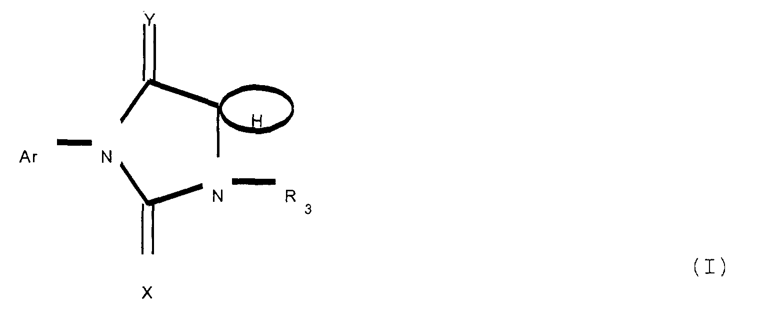 in which (Aᵣ) represents the 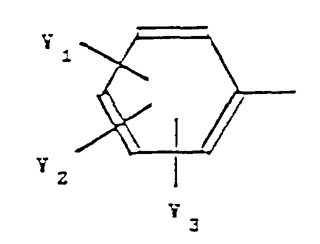 radical, (H) represents the 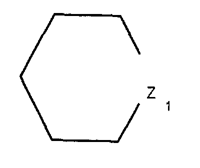
radical in which Z₁ represents an oxygen, a sulphur atom optionally oxidized in the form of sulphoxide or sulphone or an -N-R₄ radical in which R₄ is chosen from the hydrogen atom and the alkyl radicals,
W₁, W₂ and W₃, identical or different, are chosen from the hydrogen atom, the halogen atoms and the cyano, nitro, trifluoromethyl and free, esterified or salified carboxyl radicals,
R₃ is chosen from the hydrogen atom and the alkyl radicals, optionally substituted by one or more substituents chosen from the halogen atoms and the optionally salified or etherified hydroxy, alkoxy and free esterified, amidified or salified carboxy radicals,
X represents an oxygen or sulphur atom,
Y represents an oxygen atom or an NH radical,
the alkyl radicals containing at most 4 carbon atoms, it being understood that Aᵣ does not represent a non substituted phenyl radical when R3 represents a hydrogen atom,
the said products of formula (I) being in all possible racemic, enantiomic and diastereoisomic isomer forms, as well as the addition salts with mineral and organic acids or with mineral and organic bases of the said products of formula (I) .

2. The following products:
- 4-(2,4-dioxo 1-(4-hydroxybutyl) 8-oxa 1,3-diazaspiro (4.5)decan 3-yl) 2-(trifluoromethyl) benzonitrile,
- 4-(2,4-dioxo 1-(2-fluoroethyl) 8-oxa 1,3-diazaspiro (4.5)decan 3-yl) 2-(trifluoromethyl) benzonitrile,
- 3-(4-cyano 3-(trifluoromethyl) phenyl) 2,4-dioxo 8-oxa 1,3-diazaspiro(4.5)decane 1-acetonitrile,
- 4-(2,4-dioxo 1-(4-hydroxybutyl) 8-thia 1,3-diazaspiro (4.5)decan 3-yl) 2-(trifluoromethyl) benzonitrile.

3. Preparation process for the products of formula (I) as defined in claim 1, **characterized in that** a product of formula (II)
Ar-N=C=X (II)
in which X has the meaning indicated in claim 1 and Ar has the meaning indicated in claim 1 in which the optional reactive functions are optionally protected,
is reacted in the presence of a tertiary base with a product of formula (III): 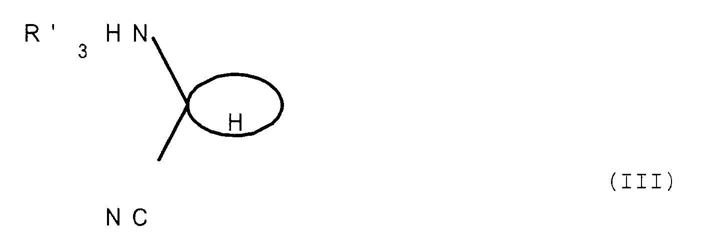 in which (H) has the meaning indicated in claim 1 and R'₃ has the meaning indicated in claim 1 for R₃, in which the optional reactive functions are optionally protected in order to obtain a product of formula (Ia): 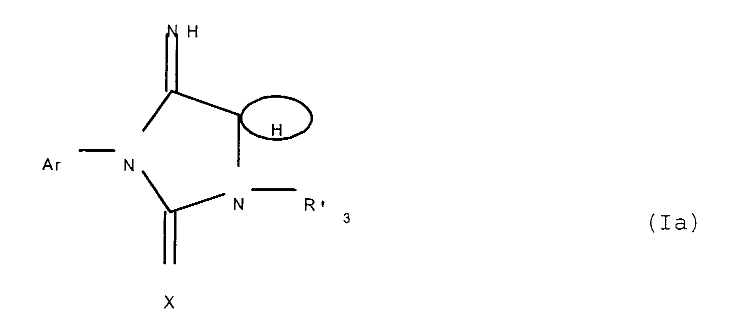 in which Ar, (H) and R'₃ have the meanings indicated above, products of formula (Ia) thus obtained, as defined above, which can, if appropriate, and if necessary or if desired, be subjected to any one or more of the following reactions, in any order:
a) elimination reaction of the optional protective groups which can be carried by R'₃, then if appropriate the action of an esterification, amidification or salification agent,
b) hydrolysis reaction of the >C=NH grouping to a carbonyl function,
c) conversion of the >C=O group to a >C=S group,
d) conversion of the >C=S group or groups to a >C=O group,
e) when R'₃ represents a alkoxyalkyl radical, conversion reaction of R'₃ to a hydroxyalkyl radical,
f) when R'₃ represents a hydrogen atom, the action of a reactive of formula Hal-R"₃ in which R"₃ has the values of R'₃ with the exception of the hydrogen value and Hal represents a halogen atom in order to obtain the products of formula (Ia), as defined above, in which R"₃ has the meaning indicated previously,
g) if desired, the action of an elimination agent of the optional protective groups which can be carried by R"₃ on the products obtained in f, or if appropriate, the action of an esterification, amidification salification agent,
h) salification reaction with a mineral or organic acid or a base in order to obtain the corresponding salt,
i) elimination reaction of the optional protective groups which can be carried by the protected reactive functions.

4. Preparation process for the products of formula (I'): 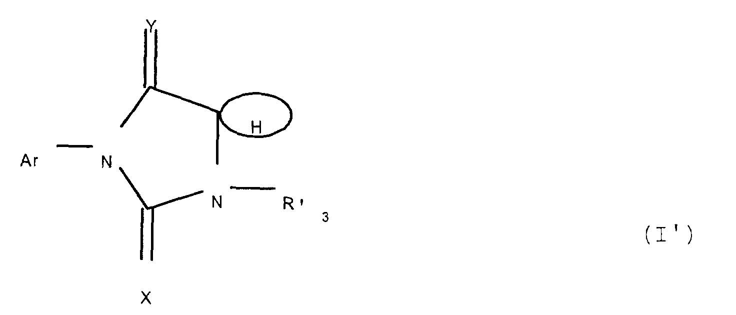 in which Ar, X, Y, R'₃ and (H) have the meanings indicated in claim 1, **characterized in that** a product of formula (VIII):
Ar-Hal (VIII)
in which Ar has the previous meaning and Hal represents a halogen atom is reacted with a product of formula (IX): 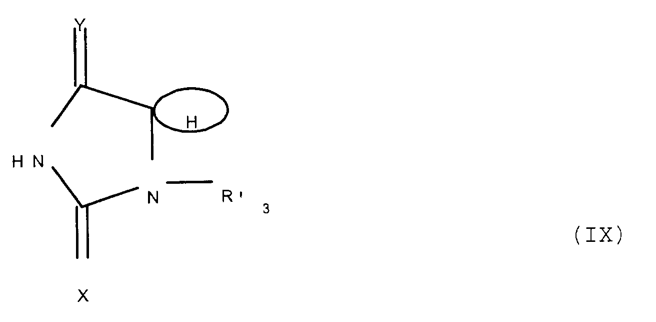 in which X, Y, R'₃ and (H) have the meanings indicated in claim 1.

5. As medicaments, the pharmaceutically acceptable products of formula (I) as defined in claim 1.

6. As medicaments, the following products:
- 4-(2,4-dioxo 1-(4-hydroxybutyl) 8-oxa 1,3-diazaspiro (4.5)decan 3-yl) 2-(trifluoromethyl) benzonitrile,
- 4-(2,4-dioxo 1-(2-fluoroethyl) 8-oxa 1,3-diazaspiro (4.5)decan 3-yl) 2-(trifluoromethyl) benzonitrile,
- 3-(4-cyano 3-(trifluoromethyl) phenyl) 2,4-dioxo 8-oxa 1,3-diazaspiro(4.5)decane 1-acetonitrile,
- 4-(2,4-dioxo 1-(4-hydroxybutyl) 8-thia 1,3-diazaspiro (4.5)decan 3-yl) 2-(trifluoromethyl) benzonitrile.

7. The pharmaceutical compositions containing, as an active ingredient, at least one of the medicaments as defined in any one of claims 5 and 6.
